(19) **Europäisches Patentamt**
**European Patent Office**
**Office européen des brevets**

(11) **EP 3 884 894 B1**

(12) **EUROPÄISCHE PATENTSCHRIFT**

(45) Veröffentlichungstag und Bekanntmachung des Hinweises auf die Patenterteilung:
**25.02.2026 Patentblatt 2026/09**

(21) Anmeldenummer: **21165392.8**

(22) Anmeldetag: **26.03.2021**

(51) Internationale Patentklassifikation (IPC):
***A61B 18/12*** *(2006.01)* ***A61B 18/14*** *(2006.01)*

(52) Gemeinsame Patentklassifikation (CPC):
**A61B 18/1206; A61B 18/149;** A61B 2018/00601;
A61B 2018/00678; A61B 2018/00767;
A61B 2018/00875; A61B 2018/00892

(54) **ELEKTROCHIRURGIE-GENERATOR, ELEKTROCHIRURGIESYSTEM UND VERFAHREN ZUM BETREIBEN EINES ELEKTROCHIRURGIE-GENERATORS**

ELECTROSURGICAL GENERATOR, ELECTROSURGICAL SYSTEM AND METHOD FOR OPERATING AN ELECTROSURGICAL GENERATOR

GÉNÉRATEUR D'ÉLECTROCHIRURGIE, SYSTÈME D'ÉLECTROCHIRURGIE ET PROCÉDÉ DE FONCTIONNEMENT D'UN GÉNÉRATEUR D'ÉLECTROCHIRURGIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.03.2020 DE 102020108614**

(43) Veröffentlichungstag der Anmeldung:
**29.09.2021 Patentblatt 2021/39**

(73) Patentinhaber: **Olympus Winter & Ibe GmbH 22045 Hamburg (DE)**

(72) Erfinder:
• **Dijkstra, Jelle
10781 Berlin (DE)**
• **Handrick, Veronika
12059 Berlin (DE)**
• **Karrasch, Andreas
12305 Berlin (DE)**
• **Breitsprecher, Frank
12527 Berlin (DE)**

(74) Vertreter: **Eisenführ Speiser
Patentanwälte Rechtsanwälte PartGmbB
Stralauer Platz 34
10243 Berlin (DE)**

(56) Entgegenhaltungen:
DE-A1- 102011 005 067    DE-A1- 102017 106 747
DE-B4- 19 827 318    DE-T2- 60 314 184
US-A1- 2004 019 351    US-A1- 2014 236 142
US-A1- 2015 272 657    US-A1- 2016 074 091

**Beschreibung**

Die Erfindung betrifft einen Elektrochirurgie-Generator, der dazu konfiguriert ist, ein elektrochirurgisches Instrument zum Plasmaschneiden von Körpergewebe mit hochfrequentem Wechselstrom zu versorgen. Die Erfindung betrifft außerdem ein Elektrochirurgiesystem, welches einen Elektrochirurgie-Generator und ein elektrochirurgisches Instrument umfasst sowie ein Verfahren zum Betreiben eines Elektrochirurgie-Generators.

Mittels Elektrochirurgie kann biologisches Gewebe - also Körpergewebe - geschnitten, koaguliert (verödet) und/oder vaporisiert werden. Für die Elektrochirurgie werden typischerweise hochfrequente Wechselströme mit einer Frequenz zwischen 0,2 MHz und 3 MHz verwendet.

Ein Elektrochirurgiesystem umfasst in der Regel einen Elektrochirurgie-Generator zum Erzeugen des hochfrequenten Wechselstroms. Der Elektrochirurgie-Generator hat in der Regel zwei Ausgänge, an die ein elektrochirurgisches Instrument angeschlossen werden kann und zwischen denen im Betrieb eine hochfrequente Wechselspannung bereitgestellt wird. Dazu umfasst ein Elektrochirurgie-Generator in der Regel ein Hochspannungs-Netzteil, das im Betrieb einen Gleichstrom erzeugt, und einen Hochfrequenz-Teil, der mit dem Hochspannungs-Netzteil verbunden ist und im Betrieb aus dem Gleichstrom einen hochfrequenten Wechselstrom erzeugt.

Elektrochirurgiesysteme werden beispielsweise in der Urologie oder Gynäkologie eingesetzt. Insbesondere kommt für eine Plasma-Vaporisation beispielsweise im Rahmen von TURis (Transurethrale Resektion in Saline) zur Behandlung der gutartigen Prostatavergrößerung in der Regel ein Elektrochirurgie-Generator in Verbindung mit einem geeigneten elektrochirurgischen Instrument, wie einem Resektoskop, zum Einsatz. Die aktive Schneid- oder Vaporisationselektrode eines solchen elektrochirurgischen Instruments befindet sich dabei in einem elektrisch leitenden Spülfluid, wie beispielsweise einer Kochsalzlösung (NaCl). Mittels der hochfrequenten Wechselspannung kann an der aktiven Elektrode des elektrochirurgischen Instruments ein Lichtbogen gezündet werden (auch Plasmazündung genannt).

Körpergewebe kann mit einem Plasma, das in einer Dampfblase - also einer Gasblase - um eine Elektrode in einer elektrisch leitenden, körperverträglichen Flüssigkeit, insbesondere einer Salzlösung erzeugt wird, vaporisiert oder geschnitten werden, indem ein Lichtbogen in einem Gasvolumen um eine aktive Elektrode des Elektrochirurgie-Instruments erzeugt wird. Dazu wird an die Elektrode eine hochfrequente Wechselspannung angelegt, die Wechselströme bewirkt, die zunächst dazu führen, dass die Salzlösung in der unmittelbaren Umgebung der aktiven Elektrode verdampft, sodass eine Dampfblase um die Elektrode entsteht. In der Dampfblase bildet sich ein elektrisches Feld zwischen der Elektrode und der flüssigen Salzlösung aus. Wenn das elektrische Feld stark genug ist, kommt es zu einer Gasentladung, auch als Lichtbogen bezeichnet, infolge einer Ionisierung des Gases in der Dampfblase, das heißt es entsteht ein Plasma (ionisiertes Gas).

Bevor das Plasma und damit ein Lichtbogen entsteht, muss in einer Initialphase eine um die Elektrode befindliche elektrisch leitende Flüssigkeit verdampft werden und anschließend in der so entstandenen Dampfblase ein Lichtbogen gezündet werden. Dies setzt voraus, dass die Dampfblase die Elektrode vollständig umgibt und keine elektrisch leitende Flüssigkeit mehr an der Elektrode anliegt, da ansonsten nicht das für eine Ionisation des Gases in der Dampfblase erforderliche starke elektrische Feld entstehen kann, denn der unmittelbare Stromfluss zwischen aktiver Elektrode und elektrisch leitender Flüssigkeit verhindert eine für die Ionisation nötige Potentialdifferenz.

Nach dem erstmaligen Zünden des Lichtbogens - also nach dem erstmaligen Entstehen von Plasma - stellt sich ein dynamisch stabiler Zustand ein, bei dem immer dort, wo die Gasblase um die Elektrode zu kollabieren droht, aufgrund der Annäherung der elektrisch leitenden Flüssigkeit an die Elektrode ein besonders starkes elektrisches Feld auftritt, welches wiederum eine besonders starke Plasmabildung und damit ein stärkeres Verdampfen der elektrisch leitenden Flüssigkeit zur Folge hat. Auf diese Weise stabilisiert sich die Gasblase um die Elektrode.

Die Initialphase, auf die schließlich die Phase eines stabilen Plasmalichtbogens folgt, umfasst somit typischerweise zwei Teilphasen:
In einer ersten Teilphase, die hier auch als Verdampfungsphase bezeichnet wird, wird die elektrisch leitende Flüssigkeit um die aktive Elektrode durch Stromfluss erhitzt und verdampft, sodass sich zunächst ein oder mehrere Gasblasen aus Dampf um die aktive Elektrode bilden, bis die aktive Elektrode vollständig von einer Gasschicht umgeben ist, die schließlich die aktive Elektrode elektrisch von der elektrisch leitenden Flüssigkeit isoliert.

Sobald dies erreicht ist, kommt es in einer zweiten Teilphase der Initialphase zum Zünden eines Lichtbogens. Diese zweite Teilphase wird hier auch als Zündphase bezeichnet. In der Zündphase bewirkt der hohe Spannungsabfall über die Gasschicht zwischen der aktiven Elektrode und der elektrisch leitenden Flüssigkeit eine Ionisation des Gases in der Dampfblase, sodass ein Plasma entsteht, das zum Schneiden oder Vaporisieren von Gewebe genutzt werden kann. Das erstmalige Zünden des Plasmas ist schwierig und hängt stark von Umgebungsbedingungen ab. In einigen Fällen entsteht ein stabiles Plasma innerhalb weniger Millisekunden. Unter ungünstigen Umständen sind jedoch mehrere Anläufe erforderlich, bevor ein stabiles Plasma gezündet wird.

Die verschiedenen Phasen und Teilphasen vor und während des Zündens des Plasmas unterscheiden sich auch hinsichtlich der vom Elektrochirurgie-Generator gelieferten elektrischen Größen.

Solange die aktive Elektrode gar nicht oder nicht vollständig von einer Gasschicht umgeben ist, ist die Impedanz zwischen der aktiven Elektrode und einer Gegenelektrode niederohmig. Die Impedanz beträgt anfänglich ungefähr 25 Ohm bis 50 Ohm, je nach Geometrie der Elektroden, der Temperatur der elektrisch leitenden Flüssigkeit usw. Die von dem Elektrochirurgie-Generator abgegebene Ausgangsspannung und der abgegebene Strom sind daher in dieser ersten Aufheizphase der die aktive Elektrode umgebenden elektrisch leitenden Flüssigkeit gleichphasig und haben keinen Gleichspannungsanteil.

Sobald die elektrisch leitende Flüssigkeit zu verdampfen beginnt und die Oberfläche der aktiven Elektrode teilweise von Gas umgeben ist, nimmt die Impedanz zwischen den Ausgängen des Elektrochirurgie-Generators zu, weil der Oberflächenwiderstand zwischen der aktiven Elektrode und der elektrisch leitenden Flüssigkeit zunimmt. Der Oberflächenwiderstand trägt jedoch nur zu einem relativ kleinen Teil zu der gesamten Impedanz zwischen den Elektroden - und damit den Ausgängen des Elektrochirurgie-Generators - bei. Daher nimmt die Impedanz während der Verdampfungsphase so lange nur geringfügig zu, bis fast die gesamte aktive Elektrode von Gas umgeben ist.

Sobald die aktive Elektrode vollständig von Gas umgeben ist, endet die Verdampfungsphase und die Zündphase beginnt. Während der Zündphase kann zwischen den Elektroden kein ohmscher Strom mehr fließen. Daher ist die Impedanz viel größer als während der Verdampfungsphase und - theoretisch - rein kapazitiv. Weil die Impedanz der elektrisch leitenden Flüssigkeit sehr gering ist, bildet die elektrisch leitende Flüssigkeit eine äquipotentiale Hülle um die Gasschicht. Über die Gasschicht zwischen der Elektrode und der elektrisch leitenden Flüssigkeit hinweg bildet sich ein elektrisches Feld aus.

Infolge des dann nicht mehr auftretenden Stromflusses wird die Gasschicht um die aktive Elektrode wieder kleiner, weil ein Teil des die Gasblase bildenden Dampfes an der Grenzschicht zwischen dem Gas und der elektrisch leitenden Flüssigkeit wieder kondensiert. Ein Dünnerwerden der Gasschicht führt dazu, dass die elektrische Feldstärke über die dünnere Gasschicht zunimmt und so groß werden kann, dass sie eine Ionisation des Gases in der Gasblase bewirkt und ein erster Lichtbogen entsteht. Die Dauer dieser Zündphase hängt von der Dicke der Gasschicht nach der Verdampfungsphase ab. Die Dauer kann sehr kurz - typischerweise im Bereich einiger Millisekunden - oder auch verschwindend kurz sein.

Sobald das elektrische Feld über die Gasschicht stark genug ist, um eine Ionisation des Gases zu bewirken, kommt es zu Plasmadurchbrüchen - das heißt Lichtbögen - und eine elektrische Entladung erfolgt. Weil die Oberfläche der aktiven Elektrode kleiner ist als die Oberfläche der die Gasblase umgebenden elektrisch leitenden Flüssigkeit, ist die elektrische Feldstärke auf Seiten der aktiven Elektrode größer als auf Seiten der elektrisch leitenden Flüssigkeit. Es kommt zu einem Gleichspannungsanteil an der Ausgangswechselspannung. Dieser Gleichspannungsanteil (DC offset) wird auch als "spark voltage" bezeichnet und beträgt etwa 100 Volt.

Infolge des elektrischen Durchbruchs (Plasmadurchbruch) verdampft Salzlösung an der Grenzfläche zwischen dem Gas und der elektrisch leitenden Flüssigkeit und die Dicke der Gasschicht nimmt am Ort des Plasmadurchbruchs wieder zu. Wann immer also die Dicke der Gasschicht infolge von Kondensation wieder so stark abnimmt, dass die elektrische Feldstärke so weit zunimmt, dass es zu einem Funkendurchschlag kommt, nimmt die Dicke der Gasschicht wegen verdampfender elektrisch leitender Flüssigkeit wieder zu. Auf diese Weise ist die Dicke der Gasschicht um die aktive Elektrode herum selbststabilisierend und das Plasma um die aktive Elektrode herum befindet sich in einer Gleichgewichtsphase. In der Gleichgewichtsphase stellt sich ein Gleichgewichtszustand ein, in dem die Dicke der Gasschicht gerade so groß ist, dass die Dickenzunahme infolge von Plasmadurchbrüchen die Dickenabnahme infolge von Kondensation an der Grenzschicht zwischen Gas und Salzlösung ausgleicht. In der Gleichgewichtsphase treten somit Plasmadurchbrüche "automatisch" dort auf, wo die Gasschicht um die aktive Elektrode am dünnsten ist, weil dort die elektrische Feldstärke am höchsten ist. Entsprechend entsteht neues Gas durch Verdampfung genau dort, wo es gebraucht wird. Die Ausgangsspannung des Elektrochirurgie-Generators bestimmt die Dicke der Gasschicht.

Für das Plasmaschneiden ist bei Elektrochirurgie-Generatoren gemäß dem Stand der Technik typischerweise eine Ausgangswechselspannung zwischen 250V und 350V, beispielsweise 280V oder 320V, vorgegeben.

US2014236142A1 offenbart einen Elektrochirurgie-Generator nach dem Stand der Technik.

In der Praxis zeigen sich gelegentlich Probleme beim Zünden des Plasmas für das Gewebeschneiden.

Ziel der Erfindung ist es, einen Elektrochirurgie-Generator hinsichtlich seiner Tauglichkeit für das Plasmaschneiden von Gewebe zu verbessern.

Die Erfindung wird durch die unabhängigen Ansprüche 1,3 und 5 definiert, weitere Ausführungsformen werden in den abhängigen Ansprüchen beschrieben.

Erfindungsgemäß wird dieses Ziel mit einem Elektrochirurgie-Generator erreicht, der dazu konfiguriert ist, im Betrieb einen hochfrequenten Wechselstrom an ein elektrochirurgisches Instrument für das Plasmaschneiden von Körpergewebe abzugeben. Der Elektrochirurgie-Generator hat Ausgänge zum Anschließen eines elektrochirurgischen Instruments, um ein an die Ausgänge angeschlossenes elektrochirurgisches Instrument mit einem hochfrequenten Wechselstrom zu versorgen.

Der Elektrochirurgie-Generator weist eine Impedanzbestimmungseinheit auf, um eine Impedanz einer an die Ausgänge angeschlossenen Last zu bestimmen. Außerdem weist der Elektrochirurgie-Generator eine Ausgangsspannungsregeleinheit auf und kann eine Spannungserfassungseinheit aufweisen.

Die Ausgangsspannungsregeleinheit ist dazu konfiguriert, eine Ausgangwechselspannung des Elektrochirurgie-Generators gemäß einem vorgegebenen Ausgangsspannungsmaximalwert zu regeln.

Die Impedanzbestimmungseinheit ist dazu ausgebildet, die Impedanz einer an den Ausgängen des Elektrochirurgie-Generators anliegenden Last zu bestimmen. Hierzu kann die Impedanzbestimmungseinheit dazu ausgebildet sein, eine Spannung, einen Strom und/oder eine Phasenverschiebung zu erfassen. Die Impedanzbestimmungseinheit kann die Impedanz beispielsweise aus dem erfassten Strom, der erfassten Spannung und ggf. der erfassten Phase bestimmen. Die erfasste Phase ist von Bedeutung, wenn eine Stromerfassungseinheit und eine Spannungserfassungseinheit nur die Effektivwerte von Strom und Spannung messen, da aus den Effektivwerten von Strom und Spannung sowie der Phasenlage die Impedanz genauso bestimmt werden kann, wie aus den Momentanwerten von Strom und Spannung.

Die Spannungserfassungseinheit ist dazu ausgebildet, die an den Ausgängen des Elektrochirurgie-Generators anliegende Spannung, insbesondere einen Gleichspannungsanteil einer an den Ausgängen anliegenden Spannung, zu erfassen.

Die Ausgangsspannungsregeleinheit ist dazu ausgebildet, die Ausgangswechselspannung - insbesondere deren Effektivwert - in Abhängigkeit von einem Ausgangsspannungsmaximalwert zu steuern. Erfindungsgemäß ist der vorgegebene Ausgangsspannungsmaximalwert zumindest für einen Teil der Initialphase ein anderer, als in der anschließenden Gleichgewichtsphase.

Gemäß einem ersten Aspekt der Erfindung ist der vorgegebene Ausgangsspannungsmaximalwert in der Verdampfungsphase - also zu Beginn der Initialphase - kleiner, als der für das eigentliche Plasmaschneiden in der Gleichgewichtsphase vorgesehene Ausgangsspannungsmaximalwert.

Gemäß einem zweiten Aspekt der Erfindung ist der vorgegebene Ausgangsspannungsmaximalwert in der Zündphase - also zum Ende der Initialphase hin - größer, als der für das eigentliche Plasmaschneiden in der Gleichgewichtsphase vorgesehene Ausgangsspannungsmaximalwert.

Beide Erfindungsaspekte können unabhängig voneinander realisiert werden und stellen somit jeweils eigene Erfindungen dar. Beide Aspekte können aber auch miteinander kombiniert werden, insbesondere dergestalt, dass der Elektrochirurgie-Generator dazu konfiguriert ist, für die Verdampfungsphase einen Ausgangsspannungsmaximalwert anzuwenden, der kleiner ist, als der für die Gleichgewichtsphase vorgesehene Ausgangsspannungsmaximalwert und für die Zündphase einen Ausgangsspannungsmaximalwert anzuwenden, der größer ist, als der für die Gleichgewichtsphase vorgesehene Ausgangsspannungsmaximalwert. Beide Aspekte tragen jeweils für sich und in Kombination miteinander zu einem zuverlässigeren Zünden eines Lichtbogens zu Beginn des Plasmaschneidens bei.

Um das Ende der Verdampfungsphase und den Beginn der Zündphase oder das Ende der Zündphase und den Beginn der Gleichgewichtsphase - oder beides - zu detektieren, ist die Ausgangsspannungsregeleinheit vorzugsweise mit der Impedanzbestimmungseinheit oder der Spannungserfassungseinheit oder beiden verbunden.

Um das Ende der Verdampfungsphase und den Beginn der Zündphase zu detektieren, kann die Ausgangsspannungsregeleinheit dazu konfiguriert sein, einen Anstieg der Impedanz an den Ausgängen des Elektrochirurgie-Generators über ein vorgegebenes Maß hinaus zu erfassen. Das vorgegebene Maß kann aus einer zu Beginn der Verdampfungsphase von der Impedanzbestimmungseinheit bestimmten Impedanz abgeleitet, sein, beispielsweise in vorgegebenes Vielfaches dieser Impedanz betragen.

Um das Ende der Zündphase und den Beginn der Gleichgewichtsphase zu detektieren kann die Ausgangsspannungsregeleinheit dazu konfiguriert sein, einen Gleichspannungsanteil an der Ausgangswechselspannung zu detektieren und den für die Gleichgewichtsphase vorgegebenen Ausgangswechselspanungsmaximalwert unmittelbar oder nach Ablauf einer vorgegebenen Zeitspanne nach Detektieren eines Gleichspannungsanteils anzuwenden.

Im Unterschied zum Stand der Technik ist für das Plasmaschneiden nicht ein einziger Ausgangswechselspannungsmaximalwert vorgegeben, der für die Gleichgewichtsphase geeignet ist und bereits in der Initialphase - also die Verdampfungsphase und die Zündphase - angewandt wird, sondern mindestens ein von dem die Gleichgewichtsphase geeigneten Ausgangswechselspannungsmaximalwert abweichender Ausgangswechselspannungsmaximalwert.

Die Erfindung schließt die Erkenntnis ein, dass ein für die Gleichgewichtsphase geeigneter Ausgangswechselspannungsmaximalwert zu instabilen Zuständen in der Initialphase führen kann, die ein zuverlässiges Zünden des Plasmas beeinträchtigen können.

Die Ausgangsspannungsregeleinheit ist somit dazu ausgebildet, die Ausgangswechselspannung in Abhängigkeit von einem Ausgangsspannungsmaximalwert zu steuern, der im Betrieb in Abhängigkeit von einem Ausgangswert der Impedanzbestimmungseinheit und/oder in Abhängigkeit von einem Ausgangswert der Spannungserfassungseinheit (anhand derer Beginn und Ende der Verdampfungs- bzw.- Zündphase detektiert werden) so vorgegeben wird, dass der Ausgangsspannungsmaximalwert in einer Verdampfungsphase eine geringere Ausgangswechselspannung vorgibt, als in einer an die Verdampfungsphase anschießenden Zündphase.

Die Ausgangsspannungsregeleinheit ist demnach so konfiguriert, dass ihr unterschiedliche Ausgangsspannungsmaximalwerte für die Initialphase und für die Gleichgewichtsphase zugrunde liegen. Die Initialphase kann dabei die Verdampfungsphase und/oder die Zündphase umfassen, wobei in der Verdampfungsphase ein unmittelbarer elektrischer Kontakt zwischen der aktiven Elektrode und der die aktive Elektrode umgebenden elektrisch leitenden Flüssigkeit besteht und elektrisch leitende Flüssigkeit durch elektrische Erwärmung der elektrisch leitenden Flüssigkeit infolge des Stromflusses durch die elektrisch leitende Flüssigkeit verdampft wird. In der Zündphase ist die aktive Elektrode vollständig von der infolge der Verdampfung entstandenen Gasblase umschlossen und die von der Impedanzbestimmungseinheit erfasste Impedanz ist wesentlich höher als während der Verdampfungsphase. Sobald es zu einer Ionisierung des Gases in der Gasblase kommt, und ein Lichtbogen entsteht, kommt es außerdem zu einem Gleichspannungsanteil in der an den Ausgängen des Elektrochirurgie-Generators anliegenden Wechselspannung. Dieser Gleichspannungsanteil wird von der Gleichspannungserfassungseinheit erfasst, sodass diese ein entsprechendes Ausgangssignal generieren kann, der ein Zeichen für einen (ersten) Lichtbogen ist.

In der Gleichgewichtsphase ist das Plasma um die aktive Elektrode stabil. Der Ausgangsspannungsmaximalwert, auf dem die Ausgangsspannungsregelung des Elektrochirurgie-Generators durch die Ausgangsspannungsregeleinheit während der Gleichgewichtsphase beruht ist typischerweise höher, als der Ausgangsspannungsmaximalwert, auf dem die Ausgangsspannungsregelung des Elektrochirurgie-Generators durch die Ausgangsspannungsregeleinheit während der Verdampfungsphase beruht.

Gemäß dem ersten Erfindungsaspekt ist der Ausgangsspannungsmaximalwert, auf dem die Ausgangsspannungsregelung des Elektrochirurgie-Generators durch die Ausgangsspannungsregeleinheit während der Verdampfungsphase beruht, niedriger, als der Ausgangsspannungsmaximalwert, der für die Ausgangsspannungsregelung des Elektrochirurgie-Generators durch die Ausgangsspannungsregeleinheit während der Gleichgewichtsphase vorgegeben ist. Vorzugsweise ist für die Verdampfungsphase kein fester Ausgangsspannungsmaximalwert vorgegeben, sondern dieser wird vorzugsweise auf Basis der während der Verdampfungsphase zwischen den Ausgängen des Elektrochirurgie-Generators erfassten Impedanz so eingestellt, dass sich am Übergang zwischen der aktiven Elektrode und der diese umgebenden Salzlösung eine annähernd konstant bleibende Stromdichte ergibt.

Gemäß dem zweiten Erfindungsaspekt ist der Ausgangsspannungsmaximalwert, auf dem die Ausgangsspannungsregelung des Elektrochirurgie-Generators durch die Ausgangsspannungsregeleinheit während der an die Verdampfungsphase anschließenden Zündphase beruht, höher, als der Ausgangsspannungsmaximalwert, der für die Ausgangsspannungsregelung des Elektrochirurgie-Generators durch die Ausgangsspannungsregeleinheit während der Gleichgewichtsphase vorgegeben ist. Ein für die Zündphase gegenüber der nachfolgenden Gleichgewichtsphase erhöhter Ausgangsspannungsmaximalwert führt zu einer erhöhten Ausgangswechselspannung des Elektrochirurgie-Generators in der Zündphase und bewirkt ein rascheres und zuverlässigeres Zünden des Plasmas.

Die Erfindung schließt die Erkenntnis ein, dass durch Messen der Ausgangsspannung und des Ausgangstroms des Elektrochirurgie-Generators ein Ableiten solcher Größen wie der Impedanz oder eines Gleichspannungsanteils und damit eine Unterscheidung zwischen den verschiedenen Phasen des Zündvorgangs möglich ist. Durch Erkennen der verschiedenen Phasen vor und während des Zündens eines Plasmas um die aktive Elektrode eines elektrochirurgischen Instruments herum ist es möglich, anstelle eines festen Ausgangsspannungsmaximalwertes für alle Phasen unterschiedliche Ausgangsspannungsmaximalwerte für jede Phase individuell gezielt einzustellen.

Vorzugsweise ist die Ausgangsspannungsregeleinheit dazu ausgebildet, die Ausgangswechselspannung in Abhängigkeit von einem Ausgangsspannungsmaximalwert zu steuern, der im Betrieb in Abhängigkeit von einem Ausgangswert der Impedanzbestimmungseinheit zum Detektieren des Beginns der Zündphase und/oder in Abhängigkeit von einem Ausgangswert der Spannungserfassungseinheit zum Detektieren des Endes der Zündphase so einzustellen, dass der Ausgangsspannungsmaximalwert in der Zündphase höher ist, als in einer an die Zündphase anschießenden Gleichgewichtsphase.

Der Elektrochirurgie-Generator ist vorzugsweise dazu ausgebildet, die Verdampfungsphase und den Beginn der Zündphase anhand einer erfassten Impedanz zu detektieren. Vorzugsweise ist der Elektrochirurgie-Generator dazu ausgebildet, als Kriterium für den Beginn der Zündphase einen Abfall der Stromstärke des Ausgangswechselstroms während der Verdampfungsphase auf einen vorgegebenen Bruchteil - beispielsweise ein Drittel - der Stromstärke des Ausgangswechselstroms zu Beginn der Verdampfungsphase anzuwenden. Der Elektrochirurgie-Generator ist somit vorzugsweise dazu ausgebildet, den Beginn der Zündphase dann zu detektieren, wenn die Stromstärke des Ausgangswechselstroms während der Verdampfungsphase auf ein Drittel oder ein Viertel der Stromstärke des Ausgangswechselstroms zu Beginn der Verdampfungsphase abgefallen ist.

Gemäß einer weiteren bevorzugten Ausführungsvariante ist der Elektrochirurgie-Generator dazu ausgebildet, ein Ende der Zündphase anhand eines Gleichspannungsanteils an einer an den Ausgängen des Elektrochirurgie-Generators anliegenden Spannung zu detektieren.

Die Ausgangsspannungsregeleinheit ist vorzugsweise dazu ausgebildet, während der Verdampfungsphase einen sich in Abhängigkeit von einem jeweils aktuellen Ausgangswert der Impedanzbestimmungseinheit und/oder der Spannungserfassungseinheit nachgeführten Ausgangsspannungsmaximalwert anzuwenden. Das Nachführen des Ausgangsspannungsmaximalwert erfolgt vorzugsweise derart, dass sich beim am Übergang von der aktiven Elektrode zu einer an dieser aktiven Elektrode anlegenden Flüssigkeit eine zumindest annähernd konstante Stromdichte ergibt. Wenn also die aktive Elektrode im Laufe der Verdampfungsphase zunehmend von einer Dampfblase bedeckt ist und somit die Fläche abnimmt, auf der die leitende Flüssigkeit die aktive Elektrode berührt, senkt die Ausgangsspannungsregeleinheit die Ausgangswechselspannung entsprechend, so dass die Stromdichte wenigstens annähernd konstant bleibt.

Vorzugsweise ist der Elektrochirurgie-Generator dazu ausgebildet, vor Beginn der Verdampfungsphase mittels der Impedanzbestimmungseinheit die Impedanz zwischen den beiden Ausgängen des Elektrochirurgie-Generators zu bestimmen und den Ausgangsspannungsmaximalwert zu Beginn der Verdampfungsphase derart einzustellen, dass die sich aus Impedanz und Ausgangsspannung ergebende Stromstärke kleiner ist, als eine maximale vom Elektrochirurgie-Generator lieferbare Stromstärke des Ausgangswechselstroms.

Alternativ kann die Ausgangsspannungsregeleinheit dazu ausgebildet sein, während der Verdampfungsphase einen für die Verdampfungsphase fest vorgegebenen Ausgangsspannungsmaximalwert anzuwenden. Dabei ist der fest vorgegebene Ausgangsspannungsmaximalwert so gewählt, dass der maximale Strom den der Elektrochirurgie-Generator liefern kann. auch zu Beginn der Verdampfungsphase bei der dann herrschen niedrigen Impedanz nicht über-schritten wird, gemäß $U_{max} = Z_{gemessen} * I_{max}$. Dadurch, dass die Impedanz Z während bis zum Ende der Verdampfungsphase nur weiter zunimmt, kann der Elektrochirurgie-Generator die vorgegebene maximale Ausgangsspannung aufrecht erhalten, da der Strom nur weiter abnimmt. Diese Variante lässt sich einfacher realisieren als ein in Abhängigkeit von einem jeweils aktuellen Ausgangswert der Impedanzbestimmungseinheit und/oder der Spannungserfassungseinheit nachgeführter Ausgangsspannungsmaximalwert und kann ebenfalls eine zu starke Dampfentwicklung zum Ende der Verdampfungsphase verhindern.

Vorzugsweise ist der Elektrochirurgie-Generator derart dimensioniert, dass er eine maximale Ausgangswechselspannung von mehr als 250 V und einen maximalen Ausgangswechselstrom von mehr als 4 A liefern kann, beispielsweise eine maximale Ausgangswechselspannung zwischen 250 V und 400 V und einen maximalen Ausgangswechselstrom zwischen 4 A und 12 A.

Erfindungsgemäß wird auch ein Elektrochirurgie-System vorgeschlagen, das eine Elektrochirurgie-Generator der hier beschriebenen Art und mit einem elektrochirurgischen Instrument aufweist, welches an Ausgänge des Elektrochirurgie-Generators angeschlossen ist oder angeschlossen werden kann. Das elektrochirurgische Instrument weist eine aktive Elektrode und wenigstens eine Gegenelektrode sowie wenigstens eine Flüssigkeitsleitung auf, die relativ zu der aktiven Elektrode so angeordnet ist, dass die aktive Elektrode im Betrieb mit einer elektrisch leitenden Flüssigkeit umspült werden kann.

Die aktive Elektrode ist vorzugsweise als Loop-Elektrode oder als Button-Elektrode ausgebildet.

Vorzugsweise ist das elektrochirurgische Instrument ein Resektoskop.

Ein weiterer Aspekt der Erfindung ist ein Verfahren zum Betreiben eines Elektrochirurgie-Generators. Eine erste Variante des Verfahrens umfasst die folgenden Schritte:

- Generieren einer Ausgangswechselspannung und Zuführen der Ausgangswechselspannung zu Ausgängen des Elektrochirurgie-Generators, wobei für die Ausgangswechselspannung ein Ausgangswechselspannungsmaximalwert vorgegeben ist,

- Bestimmen der an den Ausgängen anliegenden Last und Vergleichen der Last mit einem Grenzwert, der so gewählt ist, dass dessen Unterschreiten das Bestehen einer Verdampfungsphase anzeigt,

- wobei solange der Grenzwert unterschritten ist ein Ausgangswechselspannungsmaximalwert vorgegeben wird, der vorzugsweise mindestens 30% kleiner ist, als ein Ausgangsspannungsmaximalwert, der vorgegeben wird, wenn der Grenzwert überschritten wird, und

- Regeln der Ausgangswechselspannung auf Grundlage des jeweils vorgegebenen Ausgangswechselspannungsmaximalwertes.

Vorzugsweise wird hierbei der Ausgangswechselspannungsmaximalwert in Abhängigkeit von der bestimmten Last bei sich verändernder Last nachgeführt, solange der Grenzwert unterschritten ist.

Die Last kann beispielsweise anhand einer Impedanz an den Ausgängen des Elektrochirurgie-Generators bestimmt werden. Der Grenzwert, der als Kriterium zum Erkennen einer Verdampfungsphase dient, ist dann beispielsweise ein Impedanzwert, der ein vorgegebenes Maß definiert, das vorzugsweise aus einer zu Beginn der Verdampfungsphase von der Impedanzbestimmungseinheit erfassten Impedanz abgeleitet ist. Die Verdampfungsphase liegt vor, wenn der Grenzwert unterschritten ist, also die Impedanz das vorgegebene Maß nicht überschritten hat. Die Impedanzbestimmungseinheit bestimmt die Impedanz beispielsweise aus dem erfassten Strom, der erfassten Spannung und - falls nur die Effektivwerte des Stroms und der Spannung erfasst werden - der Phase.

Mit einem derartigen Verfahren kann eine zu starke Dampfbildung insbesondere zum Ende der Verdampfungsphase verhindert werden.

Zusätzlich oder alternativ kann das Verfahren die folgenden Schritte umfassen:

- Generieren einer Ausgangswechselspannung und Zuführen der Ausgangswechselspannung zu Ausgängen des Elektrochirurgie-Generators, wobei für die Ausgangswechselspannung ein Ausgangswechselspannungsmaximalwert vorgegeben ist, Bestimmen der an den Ausgängen (18) anliegenden Last und Vergleich der Last mit einem Grenzwert, der so gewählt ist, dass dessen Unterschreiten das Bestehen einer Verdampfungsphase anzeigt,

- Erfassen eines Gleichspannungsanteils an der an den Ausgängen anliegenden Ausgangswechselspannung,

- wobei sobald der Grenzwert überschritten wird und kein Gleichspannungsanteil an der an den Ausgängen (18) anliegenden Ausgangswechselspannung erfasst wird, ein Ausgangswechselspannungsmaximalwert vorgegeben wird, der höher ist, als ein Ausgangsspannungsmaximalwert, der vorgegeben wird, wenn ein Gleichspannungsanteil erfasst wird,, und

- Regeln der Ausgangswechselspannung auf Grundlage des Ausgangswechselspannungsmaximalwertes.

Anstelle einen Gleichspannungsanteil zu erfassen kann auch eine Frequenzanalyse der Ausgangswechselspannung erfolgen, um das Auftreten eines Lichtbogens zu detektieren. Die Frequenzanalyse kann eine Fourier-transformation insbesondere eine schnelle Fourier Transformation (FFT) umfassen.

Mit einer derartigen zweiten Variante des Verfahrens kann ein Lichtbogen nach erstmaligem Zünden stabilisiert werden.

Die beiden Verfahrensvarianten können miteinander kombiniert werden.

Vorzugsweise umfasst das Verfahren auch einen Schritt des Detektierens des Beginns einer Zündphase anhand eines Anstiegs der Impedanz über einen Grenzwert, der aus einem anfänglichen Impedanzwert und einem vorgegebenen bestimmt ist, oder anhand eines Abfalls des Ausgangswechselstroms unter einen vorgegebenen Bruchteil eines anfänglichen Ausgangswechselstromwertes.

Die Erfindung soll nun anhand von Ausführungsbeispielen mit Bezug auf die Figuren näher erläutert werden. Von den Figuren zeigt:

Figur 1: ein Elektrochirurgiesystem mit einem Elektrochirurgie-Generator und einem an diesem angeschlossenen elektrochirurgischen Instrument;

Figur 2: eine Illustration der Anwendung eines Resektoskops als elektrochirurgischem Instrument;

Figur 3: ein schematisches Schaltbild eines Elektrochirurgie-Generators;

Figur 4a)-f): verschiedene Phasen beim Zünden eines Plasmas um eine aktive Elektrode eines elektrochirurgischen Instruments;

Figur 5a)-c): eine alternative Ausführungsform einer aktiven Elektrode für ein elektrochirurgisches Instrument;

Figur 6: ein Diagramm zur Illustration des Spannungs- und des Impedanzverlaufs während der Verdampfungs- und Zündphase beim Entstehen eines Lichtbogens an der aktiven Elektrode des elektrochirurgischen Instruments;

Figur 7: ein möglicher idealer Verlauf eines Ausgangsspannungsmaximalwertes während der Verdampfungsphase, gesteuert in Abhängigkeit von der während der Verdampfungsphase erfassten Impedanz; und

Figur 8: ein möglicher vereinfachter Verlauf eines Ausgangsspannungsmaximalwertes während der Verdampfungsphase, der Zündphase und der Gleichgewichtsphase.

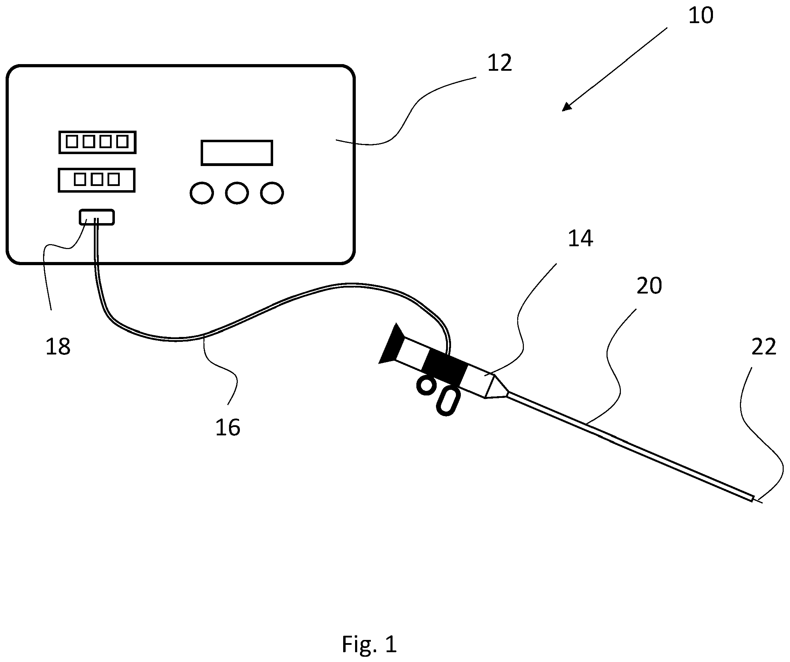

In Figur 1 ist ein Elektrochirurgiesystem 10 abgebildet. Das Elektrochirurgiesystem 10 umfasst einen Elektrochirurgie-Generator 12 und ein elektrochirurgisches Instrument 14. Das elektrochirurgisches Instrument 14 ist über ein Anschlusskabel 16 mit elektrischen Ausgängen 18 des Elektrochirurgie-Generators 12 verbunden.

In dem dargestellten Ausführungsbeispiel ist das elektrochirurgische Instrument 14 ein Resektoskop mit einem Tubus 20, durch den eine elektrisch leitende Flüssigkeit als Spülfluid zu einem distalen Ende des Tubus 20 geführt werden kann. In dem Tubus 20 ist hierzu eine Flüssigkeitsleitung vorgesehen. Die elektrisch leitende Flüssigkeit tritt am distalen Ende des Tubus 20 aus und umspült eine aktive Elektrode 22 am distalen Ende des Tubus 20. Die elektrisch leitende Flüssigkeit zirkuliert und wird durch den Tubus 20 auch wieder abgeführt. Der Tubus 20 schließt somit zumindest zwei Lumina ein, über die elektrisch leitende Flüssigkeit zum distalen Ende des Tubus 20 geführt werden kann, sodass sie dort austreten kann, während gleichzeitig Flüssigkeit vom distalen Ende des Tubus 20 durch ein weiteres Lumen wieder abgeführt wird. Die Lumina dienen somit als Flüssigkeitsleitungen. Entsprechende Schlauchanschlüsse für die elektrisch leitende Flüssigkeit sind an dem elektrochirurgischen Instrument 14 in Figur 1 nicht dargestellt.

Die aktive Elektrode 22 des elektrochirurgischen Instruments 14 kann aus dem Tubus 20 herausgeschoben sein, so wie dies in Figur 1 angedeutet ist oder sie kann in den Tubus 20 zurückgezogen sein. Typischerweise weist das elektrochirurgische Instrument 14 eine Feder auf, die entweder bewirkt, dass die aktive Elektrode 22 gegen Federkraft von Hand aus dem Tubus 22 hinausgeschoben werden muss oder umgekehrt, gegen die Federkraft von Hand in den Tubus 22 zurückgezogen werden kann.

Die aktive Elektrode 22 kann verschiedene Formen haben, beispielsweise eine Knopfelektrode (Button-Elektrode) oder auch eine Schlaufen-Elektrode sein. In Figur 1 nicht dargestellt ist eine Gegenelektrode, die beispielsweise von dem Tubus 20 gebildet sein kann, aber auch zusammen mit der aktive Elektrode 22 aus dem Tubus 20 hervorgeschoben oder in diesen zurückgezogen sein kann.

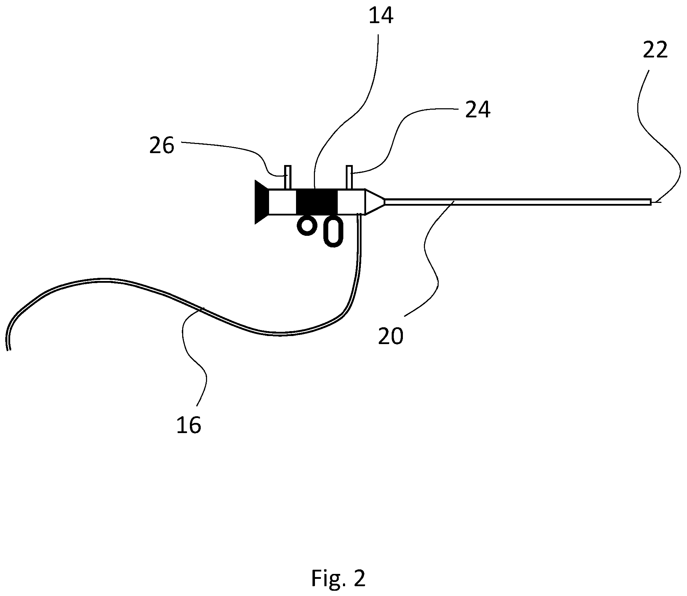

Figur 2 zeigt ein elektrochirurgisches Instrument 14 in der Anwendung. Der Figur 2 sind neben dem Anschlusskabel 16 auch Schläuche 24 und 26 zu entnehmen, die dem Zu- und Abführen der elektrisch leitenden Flüssigkeit dienen. Am distalen Ende des Tubus 20 des elektrochirurgischen Instruments 14 ist eine aktive Elektrode 22 aus dem Tubus 20 ausgefahren.

Wenn zwischen der aktive Elektrode 22 und einer entsprechenden Gegenelektrode eine hochfrequente Wechselspannung angelegt wird, bewirkt diese zunächst einen Stromfluss durch die, die aktive Elektrode 22 umgebende, elektrisch leitende Flüssigkeit. Infolge des Stromflusses erwärmt sich die elektrisch leitende Flüssigkeit um die aktive Elektrode 22 herum und verdampft im Ergebnis. Hierbei fließt solange Strom zwischen der aktiven Elektrode 22 in der entsprechenden Gegenelektrode durch die elektrisch leitende Flüssigkeit, bis die aktive Elektrode 22 vollständig von einer Dampfblase umgeben ist. Sobald dies der Fall ist, bildet sich in der so entstandenen Dampfblase ein elektrisches Wechselfeld zwischen der aktiven Elektrode 22 und der die Dampfblase umhüllenden elektrisch leitenden Flüssigkeit aus. Wenn die Feldstärke des elektrischen Wechselfelds groß genug ist, kommt es zu einer Ionisierung des Gases in der Dampfblase um die aktive Elektrode 22 und ein Plasma - erkennbar an einem Lichtbogen - entsteht. Dabei entsteht der erste Lichtbogen dort, wo die Dampfblase um die, die aktive Elektrode umgebende, elektrisch leitende Flüssigkeit der aktiven Elektrode 22 am nächsten kommt, da dort die Feldstärke des elektrischen Wechselfeldes am größten ist. Der so entstehende Lichtbogen bewirkt, dass weitere Flüssigkeit der elektrisch leitenden Flüssigkeit verdampft, sodass die Dampfblase um die aktive Elektrode 22 im Ergebnis nicht kollabiert, sondern sich ein dynamisch stabiler Gleichgewichtszustand um die aktive Elektrode 22 ausbildet.

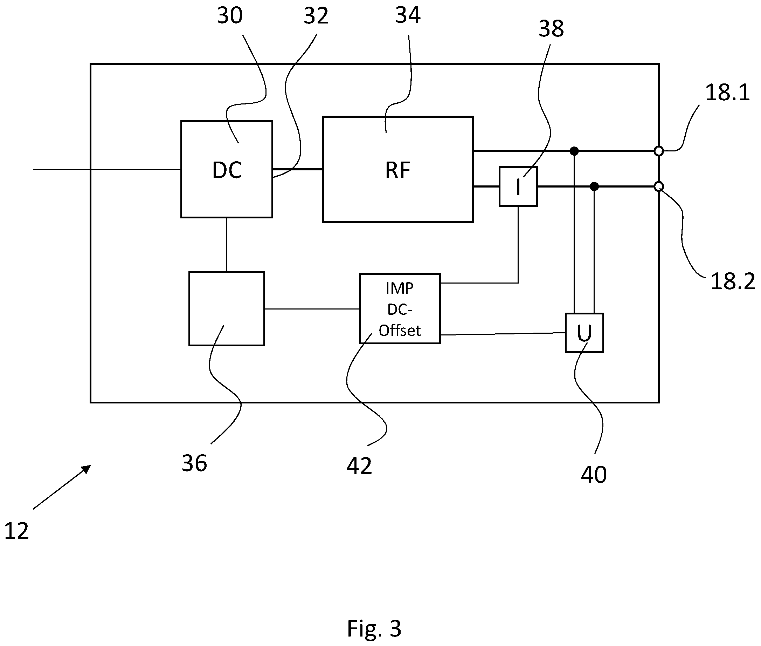

Die zum Erzeugen und Aufrechterhalten eines Plasmas um die aktive Elektrode 22 benötigte Wechselspannung - und ebenso der zum Verdampfen der elektrisch leitenden Flüssigkeit benötigte Wechselstrom - werden von dem Elektrochirurgie-Generator 12 bereitgestellt. Wie Figur 3 zu entnehmen ist, weist der Elektrochirurgie-Generator 12 hierzu ein Hochspannungsnetzteil 30 auf, das beispielsweise an das übliche öffentliche Stromversorgungsnetz angeschlossen werden kann und an seinem Ausgang 32 einen Hochspannungsgleichstrom bereitstellt. Dieser Hochspannungsgleichstrom wird einem Hochfrequenzteil 34 des Elektrochirurgie-Generators 12 zugeführt. Der Hochfrequenzteil 34 des Elektrochirurgie-Generators 12 wirkt als Wechselrichter und erzeugt eine hochfrequente Wechselspannung, die über einen Ausgangstransformator (nicht dargestellt) des Hochfrequenzteils 34 an Ausgänge 18.1 und 18.2 des Elektrochirurgie-Generators 12 abgegeben wird. An die Ausgänge 18.1 und 18.2 des Elektrochirurgie-Generators 12 kann das elektrochirurgische Instrument 14 angeschlossen werden, wie es in Figur 1 dargestellt ist.

Um die Ausgangsspannung des Elektrochirurgie-Generators 12 zu steuern, ist eine Ausgangsspannungsregeleinheit 36 vorgesehen, die die Ausgangsspannung an den Ausgängen 18.1 und 18.2 auf Basis eines Ausgangsspannungsmaximalwertes derart gesteuert, dass ein eingestellter Ausgangsspannungsmaximalwert im Betrieb nicht überschritten wird. Erfindungsgemäß ist vorgesehen, dass der jeweils anzuwendende Ausgangsspannungsmaximalwert für die verschiedenen Phasen während des Zündens des Plasmas verschieden vorgegeben sein soll, wobei die Phasen zum Zünden des Plasmas anhand der elektrischen Ausgangswerten an den Ausgängen 18.1 und 18.2 erkannt werden sollen.

Hierzu sind eine Stromerfassungseinheit 38 und eine Spannungserfassungseinheit 40 vorgesehen, die jeweils den über die beiden Ausgänge 18.1 und 18.2 abgegebenen Strom und die gleichzeitig über die beiden Ausgänge 18.1 und 18.2 abfallende Spannung erfassen. Die Ausgangswerte der Stromerfassungseinheit 38 und der Spannungserfassungseinheit 40 werden einer Auswerteeinheit 42 zugeführt, die dazu konfiguriert ist, zum einen die Impedanz einer an den Ausgängen 18.1 und 18.2 anliegenden Last zu bestimmen. In dem Sinne ist die Auswerteeinheit 42 eine Impedanzbestimmungseinheit. Die Impedanzbestimmungseinheit kann die Impedanz beispielsweise aus dem erfassten Strom, der erfassten Spannung und ggf. der erfassten Phase bestimmen. Die erfasste Phase ist von Bedeutung, wenn eine Stromerfassungseinheit und eine Spannungserfassungseinheit nur die Effektivwerte von Strom und Spannung messen, da aus den Effektivwerten von Strom und Spannung sowie der Phasenlage die Impedanz genauso bestimmt werden kann, wie aus den Momentanwerten von Strom und Spannung. Zum anderen ist die Auswerteeinheit 42 auch dazu konfiguriert, einen Gleichspannungsanteil an der über die Ausgänge 18.1 und 18.2 abfallenden Wechselspannung zu erfassen. In diesem Sinne dient die Auswerteeinheit 42 auch als Gleichspannungserfassungseinheit. Die Auswerteeinheit 42 liefert von der erfassten Impedanz und dem erfassten Gleichspannungsanteil abhängige Ausgangswerte an die Ausgangsspannungsregeleinheit 36. Die Ausgangsspannungsregeleinheit 36 ist dazu ausgebildet, einen jeweiligen der Ausgangsspannungsregelung zugrundeliegenden Ausgangsspannungsmaximalwert auf Basis der von der Auswerteeinheit 42 gelieferten Ausgangswerte für die Impedanz und den Gleichspannungsanteil zu bestimmen.

Die Steuerung der Ausgangsspannung des Elektrochirurgie-Generators 12 erfolgt somit durch Bilden entsprechender Ausgangsspannungsmaximalwerte für die Ausgangsspannungsregeleinheit 36.

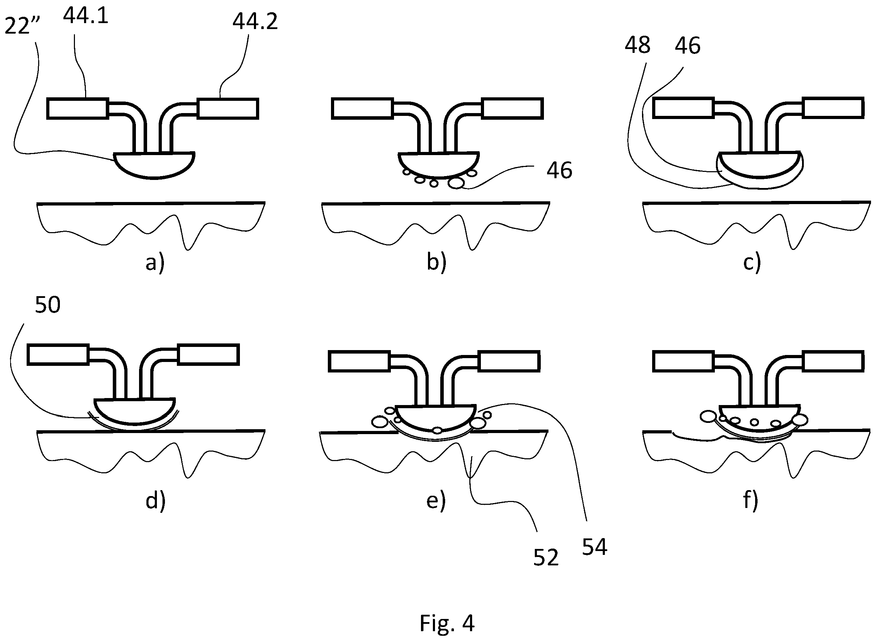

In Figur 4 sind die verschiedenen Phasen beim Zünden eines Plasmas um eine aktive Elektrode - in diesem Fall eine Knopfelektrode 22' - schematisch dargestellt.

Um Zuleitungen 42 zu der aktiven Elektrode 22' herum sind im Vergleich zur aktiven Elektrode 22' großflächigere Gegenelektroden 44.1 und 44.2 vorgesehen. Bei der Anwendung sind sowohl die aktive Elektrode 22' als auch die Gegenelektroden 44.1 und 44.2 von einer leitenden Kühlflüssigkeit, nämlich von einer Kochsalzlösung, umgeben. Wenn zwischen der aktiven Elektrode 22' und den Gegenelektroden 44.1 und 44.2 eine hochfrequente Wechselspannung aus dem Generator 12 angelegt wird, fließt zunächst ein Wechselstrom durch die, die aktive Elektrode 22 und die Gegenelektroden 44.1 und 44.2 umgebende Leitflüssigkeit. Dies ist in Figur 4a angedeutet. Durch den Stromfluss erwärmt sich die leitende Flüssigkeit und es entstehend dann Blasen 46, wie in Figur 4b angedeutet ist. Sobald die aktive Elektrode 22' vollständig von der Dampfblase 46 umgeben ist, kommt es zu keinem direkten Stromfluss zwischen der aktiven Elektrode 22' und der leitenden Flüssigkeit. Vielmehr bildet sich zwischen der aktiven Elektrode 22' auf der einen Seite und der Grenzschicht 48 zwischen der leitenden Flüssigkeit und der Dampfblase 46 auf der anderen Seite ein elektrisches Wechselfeld aus. Die Feldstärke dieses elektrischen Wechselfeldes ist umso größer, je dünner die Dampfblase 46 ist. Dort, wo die leitende Flüssigkeit bzw. die Grenzschicht 48 der aktiven Elektrode 22' am nächsten kommt, ist die elektrische Feldstärke am größten. Die vollständig ausgebildete Dampfblase 46 ist in Figur 4c dargestellt.

Wenn die elektrische Feldstärke zwischen der aktiven Elektrode 22' und der die Dampfblase 46 umgebenden elektrisch leitenden Flüssigkeit ein entsprechendes Maß überschreitet, kommt es zu einer Ionisation des Gases in der Dampfblase 46 und ein Plasma 50 - erkennbar an einem Lichtbogen - entsteht um die aktive Elektrode 22'. Sobald das Plasma gezündet ist, kann die aktive Elektrode 22 in die Nähe von zu behandelndem biologischen Gewebe 50 gebracht werden, um mithilfe des Plasmas 50 einen Teil des biologischen Gewebes 52 zu verdampfen und das biologische Gewebe 52 so teilweise abzutragen oder zu schneiden. Dies in den Figuren 4d und 4e dargestellt.

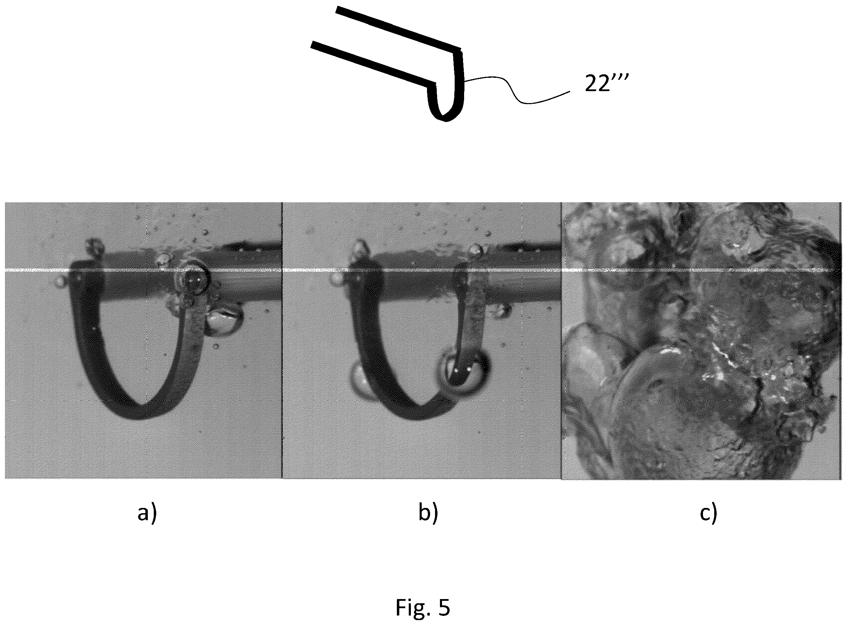

Um biologisches Gewebe wie mit einem Schälmesser zu schneiden, kann auch eine Schlaufenelektrode 22" als aktive Elektrode vorgesehen sein. Dies ist in Figur 5 dargestellt.

Während der Verdampfungsphase, wie sie beispielsweise in Figur 4b angedeutet ist, ist sukzessive ein immer größerer Teil der aktiven Elektrode 22 von einer Dampfblase 46 umgeben, sodass die Kontaktfläche zwischen der die aktive Elektrode 22 umgebenden leitenden Flüssigkeit und der aktiven Elektrode 22 immer kleiner wird. Da die Impedanz zwischen der aktiven Elektrode 22 und den Gegenelektroden 44.1 und 44.2 nicht im gleichen Maße sinkt, wie die Berührungsfläche zwischen der elektrisch leitenden Flüssigkeit und der aktiven Elektrode 22 abnimmt, erhöht sich bei gleichbleibender Ausgangsspannung des Elektrochirurgie-Generators 12 die Stromdichte in der elektrisch leitenden Flüssigkeit dort, wo diese die aktive Elektrode 22 berührt. Der Grund hierfür ist, dass die Impedanz zwischen der aktiven Elektrode 22 und den Gegenelektroden 44.1 und 44.2 nicht allein vom Übergangswiderstand von der aktiven Elektrode 22 zur elektrisch leitenden Flüssigkeit bestimmt ist, sondern auch vom Widerstand (oder der Impedanz) der elektrisch leitenden Flüssigkeit und dem Übergangswiderstand zu den Gegenelektroden 44.1 und 44.2. Beispielsweise kann angenommen werden, dass die Impedanz zwischen der aktiven Elektrode 22 und den Gegenelektroden 44.1 und 44.2 anfänglich in der Verdampfungsphase (siehe Figur 4a) etwa 25 $\Omega$ beträgt. Diese 25 $\Omega$ setzen sich beispielsweise aus etwa 10 $\Omega$ Übergangswiderstand zwischen der aktiven Elektrode 22 und der elektrisch leitenden Flüssigkeit zusammen sowie etwa 15 $\Omega$ Impedanz der elektrisch leitenden Flüssigkeit (einschließlich des Übergangswiderstands zwischen der elektrisch leitenden Flüssigkeit und den Gegenelektroden 44.1 und 44.2). In diesem Beispiel sind die 15 $\Omega$ Impedanz der elektrisch leitenden Flüssigkeit als mehr oder weniger konstant anzunehmen und ändern sich nicht, wenn die Dampfblase 46 um die aktive Elektrode 22 größer wird. Der Übergangswiderstand zwischen der aktiven Elektrode 22 und der elektrisch leitenden Flüssigkeit steigt hingegen - und zwar umgekehrt proportional zu dem Bedeckungsgrad, mit dem die Dampfblase die aktive Elektrode 22 bedeckt. Ein Bedeckungsgrad von 0 bedeutet hierbei, dass noch keine Dampfblase entstanden ist und die elektrisch leitende Flüssigkeit die aktive Elektrode 22 vollflächig berührt. Ein Bedeckungsgrad von 1 bedeutet, dass die aktive Elektrode 22 vollständig von einer Dampfblase 46 umgeben ist. Unter dieser Annahme ergibt sich, dass die Impedanz zwischen der aktiven Elektrode 22 und den Gegenelektroden 44.1 und 44.2 gemäß folgender Formel von dem Bedeckungsgrad abhängt:

$$Z = 15\,\Omega + \frac{10\,\Omega}{(1-sc)}$$

wobei Z die Impedanz zwischen der aktiven Elektrode 22 und den Gegenelektroden 44.1 und 44.2 ist und sc der Bedeckungsgrad ist, mit dem die Dampfblase 46 die Oberfläche der aktiven Elektrode 22 bedeckt. Wie bereits gesagt, bedeutet ein Bedeckungsgrad sc = 0, dass die elektrisch leitende Flüssigkeit die Oberfläche der aktiven Elektrode 22 vollflächig berührt, während ein Bedeckungsgrad sc = 1 bedeutet, dass die Oberfläche der aktiven Elektrode 22 vollständig in eine Dampfblase 46 eingehüllt ist.

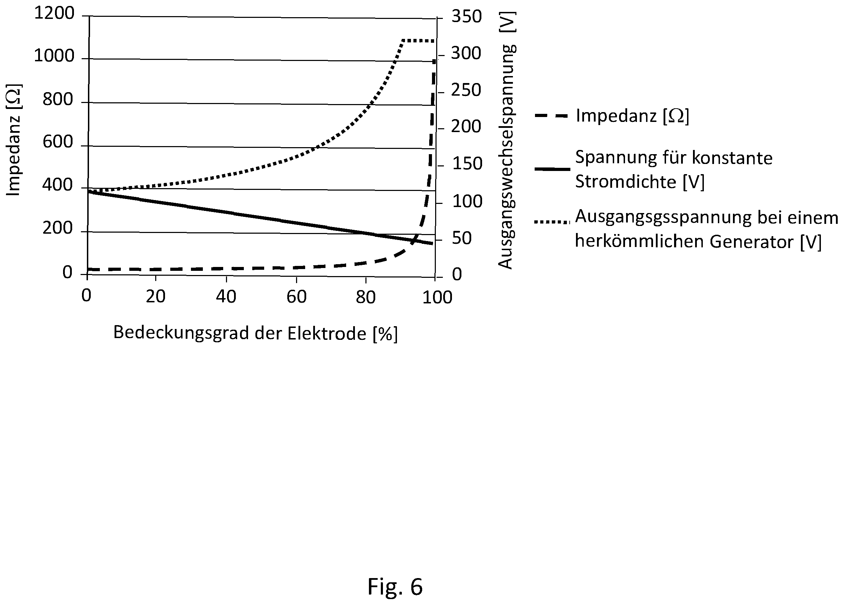

Das Ergebnis ist, dass die Impedanz in der Anfangsphase der Verdampfungsphase zunächst nur langsam ansteigt und zum Ende der Verdampfungsphase beispielsweise bei einem Bedeckungsgrad in der Größenordnung von 0,8 (80%) stark ansteigt. Mit einem derartigen Anstieg der Impedanz geht zumindest anfänglich auch ein Anstieg der Ausgangsspannung einher, weil der Elektrochirurgie-Generator 12 nur einen begrenzten Maximalstrom liefern kann, so das der Elektrochirurgie-Generator 12 seine maximale Ausgangsspannung von beispielsweise 320 V$_{effektiv}$ bei geringer Lastimpedanz nicht erreichen kann. Dies ist in Figur 6 durch eine gepunktete Linie angedeutet.

Hintergrund ist, dass ein typischer Elektrochirurgie-Generator 12 nur einen begrenzten maximalen Ausgangsstrom von beispielsweise 4 bis 5 A$_{effektiv}$ liefern kann, so dass eine vorgegebene maximale Ausgangsspannung von beispielsweise 320 V$_{effektiv}$ oder 350 V$_{effektiv}$ bei niedrigen Impedanzwerten nicht erreicht werden kann. Bei einer anfänglich niedrigen Impedanz zwischen der aktiven Elektrode 22 und den Gegenelektroden 44.1 und 44.2 - und damit auch zwischen den Ausgängen 18.1 und 18.2 des Elektrochirurgie-Generators 12 - von etwa 25 Ω kommt der begrenzte maximale Ausgangsstrom des Elektrochirurgie-Generators 12 als begrenzende Größe zum Tragen, sodass der Elektrochirurgie-Generator 12 nicht seine maximale Ausgangsspannung abgeben kann. Wäre dies möglich, würde der Elektrochirurgie-Generator 12 bei einer Ausgangsspannung von 320 V$_{effektiv}$ einen Strom von 12,8 A$_{effektiv}$ liefern, wenn die Last 25 Ω beträgt.

Mit zunehmendem Bedeckungsgrad der aktiven Elektrode 22 steigt somit die Ausgangsspannung des Elektrochirurgie-Generators 12, während der von dem Elektrochirurgie-Generator 12 abgegebene Strom konstant bleibt. Damit steigen mit zunehmendem Bedeckungsgrad sc sowohl die Ausgangsspannung, die Ausgangsleitung und auch die Stromdichte am Übergang zwischen der aktiven Elektrode 22 und der elektrisch leitenden Flüssigkeit. Erst wenn die Impedanz zwischen der aktiven Elektrode 22 und den Gegenelektroden 44.1 und 44.2 infolge der größer werdenden Dampfblase so groß ist, dass der Elektrochirurgie-Generator 12 seine maximale Ausgangsspannung von beispielsweise 320 V$_{ef-fektiv}$ erreicht, nimmt die Stromstärke des von dem Elektrochirurgie-Generators 12 abgegebenen Stroms ab. Dies ist jedoch erst ganz am Ende der Verdampfungsphase der Fall - bei dem in Figur 6 abgebildeten Beispiel beispielsweise bei einem Bedeckungsgrad von etwa 0,9 (90 %).

Die mit zunehmenden Bedeckungsgrad zunehmende Stromdichte hat zur Folge, dass sich die, die aktive Elektrode 22 berührende elektrisch leitende Flüssigkeit immer schneller erwärmt, sodass es zu schlagartigen, fast schon explosionsartigen Verdampfungen kommen kann, wie dies in Figur 5c zu sehen ist.

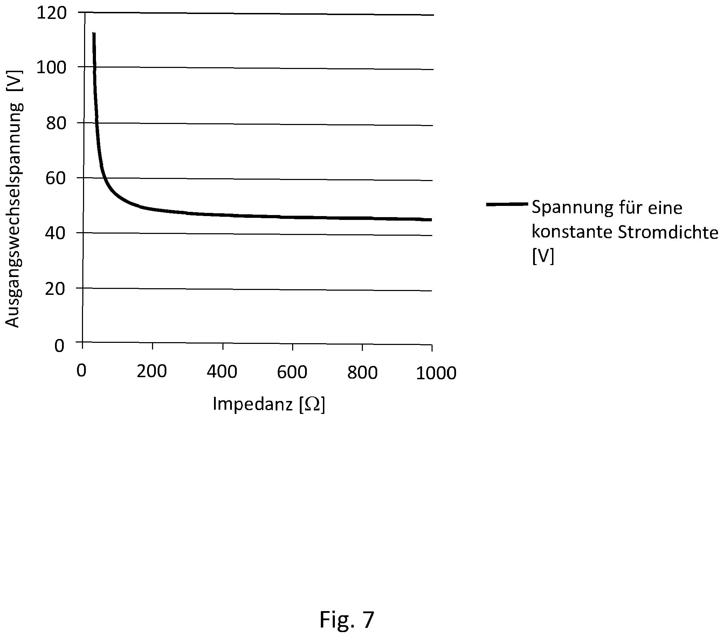

Dieses Verhalten ist unerwünscht und kann ein zuverlässiges und schnelles Zünden eines Plasmas beeinträchtigen. Daher ist erfindungsgemäß vorgesehen, dass die maximale Ausgangsspannung des Elektrochirurgie-Generators 12 während der Verdampfungsphase unter einen Wert abgesenkt wird, der als Ausgangsspannungsmaximalwert für die spätere Gleichgewichtsphase vorgesehen ist. Dies geschieht, indem ein Ausgangsspannungsmaximalwert für die Verdampfungsphase so gebildet wird, dass die maximale Ausgangsspannung des Elektrochirurgie-Generators 12 entsprechend niedrig ist. Idealerweise wird der Ausgangsspannungsmaximalwert für die maximale Ausgangsspannung des Elektrochirurgie-Generators 12 gemäß dem Bedeckungsgrad nachgeführt, sodass die maximale Ausgangsspannung bei einem niedrigen Bedeckungsgrad zunächst größer ist und dann mit zunehmendem Bedeckungsgrad abnimmt, sodass sich die durch die Ausgangsspannung des Elektrochirurgie-Generators 12 bewirkte Stromstärke umgekehrt proportional zum Bedeckungsgrad der Elektrode verringert und im Ergebnis eine annähernd konstante Stromdichte erzielt wird. Da der Bedeckungsgrad sc praktisch nicht unmittelbar bestimmt oder gemessen werden kann, wird gemäß einer bevorzugten Ausführungsvariante die zwischen den Ausgängen 18.1 und 18.2 herrschende Impedanz - die ja, wie zuvor dargelegt, von dem Bedeckungsgrad sc abhängt - herangezogen, um einen geeigneten Ausgangsspannungsmaximalwert während der Verdampfungsphase zu bilden und nachzuführen. Figur 7 zeigt, wie in dem hier dargestellten Beispiel der Ausgangsspannungsmaximalwert von der erfassten Impedanz zwischen Ausgängen 18.1 und 18.2 abhängen kann, damit sich unter den zuvor genannten Annahmen (Impedanz der leitenden Flüssigkeit von etwa 15 Ω) eine konstante Stromdichte während der gesamten Verdampfungsphase ergibt.

Gemäß einer alternativen Ausführungsvariante wird der Ausgangsspannungsmaximalwert während der Verdampfungsphase nicht kontinuierlich in Abhängigkeit von dem Bedeckungsgrad oder ersatzweise in Abhängigkeit von der erfassten Impedanz nachgeführt, sondern auf einen konstanten Wert für die Verdampfungsphase festgelegt, wobei die Verdampfungsphase nach wie vor anhand des anliegenden Impedanzwertes erkannt wird.

Es sei darauf hingewiesen, dass Figur 6 einen im Beispielsfall idealen Verlauf der Ausgangswechselspannung während der Verdampfungsphase in Abhängigkeit von dem Bedeckungsgrad illustriert, und zwar durch die in Figur 6 eingezeichnete rote Linie. Demgemäß beträgt der Effektivwert der Ausgangswechselspannung zu Beginn der Verdampfungsphase zwischen 110 und 120 V und zum Ende der Verdampfungsphase knapp 50 V. Ersatzweise kann aber auch ein konstanter Effektivwert für die Ausgangswechselspannung des Elektrochirurgie-Generators 12 während der Verdampfungsphase vorgegeben werden, wobei dieser konstante Wert beispielsweise zwischen 50 und 120 V oder besser noch zwischen 80 und 100 $V_{effektiv}$ liegen kann.

Wie ebenfalls eingangs erläutert, führt das Plasma um die aktive Elektrode 22 dazu, dass es zu einem Gleichspannungsanteil (*DC offset* oder auch *spark voltage* genannt) in der Ausgangswechselspannung des Elektrochirurgie-Generators 12 kommt, sobald das Plasma gezündet hat. Entsprechend kann das erfolgreiche Zünden des Plasmas an dem Auftreten eines Gleichspannungsanteils an der Ausgangswechselspannung des Elektrochirurgie-Generators 12 erkannt werden.

Das Ende der Verdampfungsphase und damit der Beginn der Zündphase wird vorzugsweise anhand eines Anstiegs der Impedanz über einen aus einem zu Beginn der Verdampfungsphase bestimmten Impedanzwert und einem vorgegebenen Faktor bestimmten Detektions-Impedanzwert oder anhand eines Abfalls des Ausgangswechselstroms unter einen vorgegebenen Bruchteil eines zu Beginn der Verdampfungsphase bestimmten Ausgangswechselstromwertes detektiert werden. Mit Detektieren des Beginns der Zündphase wird die Ausgangswechselspannung des Elektrochirurgie-Generators 12 auf ein möglichst hohes Maß erhöht wird, das größer ist als diejenige Ausgangswechselspannung, die für die spätere Gleichgewichtsphase vorgesehen ist. Damit kann ein sicheres Zünden des Lichtbogens gewährleistet werden. Sobald ein erster Lichtbogen entsteht - und somit auch ein Gleichspannungsanteil in der Ausgangswechselspannung des Elektrochirurgie-Generators 12 erfasst wird - ist es wünschenswert, dass die Ausgangswechselspannung des Elektrochirurgie-Generators 12 für eine bestimmte Zeit auf dem erhöhten Ausgangswechselspannungswert gehalten wird, der größer ist als diejenige Ausgangswechselspannung, die für die spätere Gleichgewichtsphase vorgesehen ist. Die bestimmte Zeit, während der die Ausgangswechselspannung des Elektrochirurgie-Generators 12 auf dem erhöhten Ausgangswechselspannungswert gehalten wird kann beispielsweise 10 bis 80 ms oder 40 bis 60 ms betragen.

Entsprechend ist es gemäß einer bevorzugten Ausführungsvariante vorgesehen, dass die Ausgangsspannungsregeleinheit den Ausgangsspannungsmaximalwert auf einen über den für die Gleichgewichtsphase vorgesehenen Spannungswert erhöht. Der für die für die Gleichgewichtsphase vorgesehene Ausgangsspannungswert beträgt beispielsweise zwischen 250 $V_{effektiv}$ und 320 $V_{effektiv}$. Der erhöhte Ausgangswechselspannungswert kann dann beispielsweise 300 $V_{effektiv}$ bis 350 $V_{effektiv}$ betragen. Eine derartig erhöhte Ausgangsspannung des Elektrochirurgie-Generators 12 während der Zündphase führt dazu, dass der Lichtbogen möglichst zuverlässig aufrechterhalten wird, bis sich das Plasma in der Dampfblase um die aktive Elektrode 22 stabilisiert hat. Sobald dies der Fall ist - und somit die eingangs genannte Gleichgewichtsphase beginnt - kann die Ausgangsspannung des Elektrochirurgie-Generators 12 durch entsprechendes Absenken des Ausgangsspannungsmaximalwertes für die Ausgangsspannungsregeleinheit 36 wieder gesenkt werden. Dies kann allmählich oder schlagartig erfolgen. Durch Senken der Ausgangswechselspannung des Elektrochirurgie-Generators 12 nach Zünden des Plasmas wird die Plasmaschicht (Dampfblase) um die aktive Elektrode 22 etwas dünner, jedoch ohne, dass das Plasma erlischt, solang die Ausgangswechselspannung des Elektrochirurgie-Generators ausreichend hoch bleibt und beispielsweise 280 bis 300 V beträgt.

In einem einfachen Ausführungsbeispiel könnte der Elektrochirurgie-Generator 12 und dessen Ausgangsspannungsregeleinheit 36 so ausgebildet sein, dass der Elektrochirurgie-Generator 12 anfänglich eine maximale Ausgangswechselspannung zwischen 100 und 200 V so lange abgibt, bis eine Gleichspannungserfassungseinheit einen Gleichspannungsanteil in der Ausgangswechselspannung des Elektrochirurgie-Generators 12 erfasst, der größer als beispielsweise 50 Ω ist. Sobald ein derartiger Gleichspannungsanteil in der Ausgangswechselspannung des Elektrochirurgie-Generators 12 erfasst wird, schaltet die Ausgangsspannungsregeleinheit 36 auf einen anderen Ausgangsspannungsmaximalwert von beispielsweise 320 V um, um ein Plasma in der Zündphase stabil zu halten. Der erhöhte Ausgangsspannungsmaximalwert für die Zündphase kann beispielsweise nach einer vorgegebenen Zeit von 10 bis 50 ms wieder auf einen etwas niedrigeren Wert für die Ausgangswechselspannung abgesenkt werden, beispielsweise auf einen Ausgangsspannungsmaximalwert der zwischen 250 und 300 $V_{effektiv}$ liegt.

Mit einem derartigen Elektrochirurgie-Generator 12 ist es möglich, ein Plasma um eine aktive Elektrode 22 zuverlässig und weniger abhängig von Umgebungsparametern innerhalb einer kurzen, nicht allzu stark schwankenden Zeit zu zünden. Dies erleichtert einem Chirurgen den Gebrauch eines entsprechenden Elektrochirurgiesystems 10 sehr.

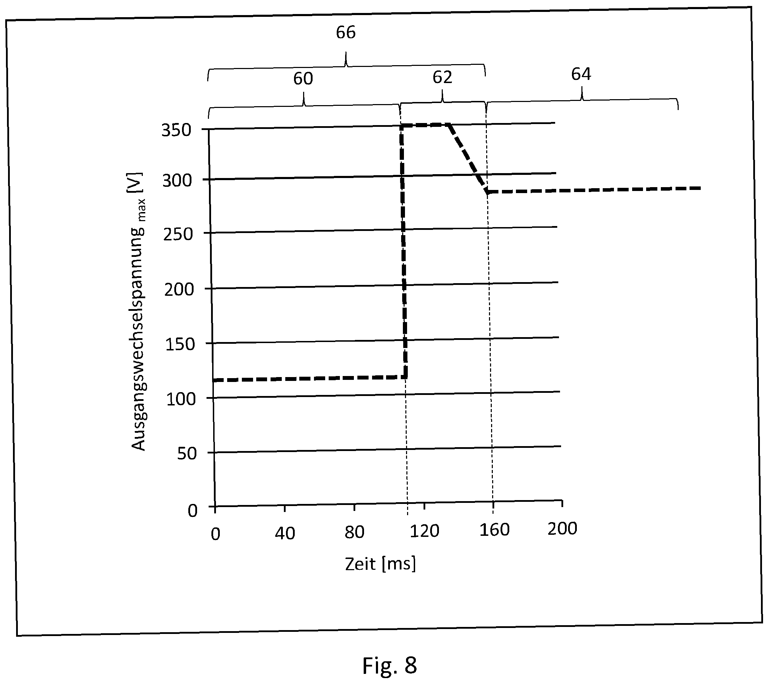

Figur 8 illustriert einen möglichen vereinfachten Verlauf eines Ausgangsspannungsmaximalwertes während der Verdampfungsphase 60, der Zündphase 62 und der Gleichgewichtsphase 64. Während der Verdampfungsphase 60 beträgt der Ausgangsspannungsmaximalwert beispielsweise 100 $V_{effektiv}$ oder 120 $V_{effektiv}$.

Zu Beginn der Zündphase 62 wird der Ausgangsspannungsmaximalwert dann beispielsweise auf 300 $V_{effektiv}$ oder 350 $V_{effektiv}$ erhöht. Nach Ende der Zündphase 62 wird der Ausgangsspannungsmaximalwert dann auf den für die Gleichgewichtsphase 64 vorgesehenen Ausgangsspannungsmaximalwert von beispielsweise 280 $V_{effektiv}$ oder 320 $V_{effektiv}$ abgesenkt. Die Verdampfungsphase 60 und die Zündphase 62 bilden zusammen die Initialphase 66, während der der Ausgangsspannungsmaximalwert entweder kleiner oder größer ist als während der sich anschließenden Gleichgewichtsphase 64.

Bezugszeichen

| 10 | Elektrochirurgiesystem |
|---|---|
| 12 | Elektrochirurgie-Generator |
| 14 | elektrochirurgisches Instrument |
| 16 | Anschlusskabel |
| 18, 18.1, 18.2 | Ausgänge des Elektrochirurgie-Generators |
| 20 | Tubus |
| 22, 22' | aktive Elektrode |
| 24, 26 | Schläuche |
| 30 | Hochspannungsnetzteil |
| 34 | Hochfrequenzteil |
| 36 | Ausgangsspannungsregeleinheit |
| 38 | Stromerfassungseinheit |
| 40 | Spannungserfassungseinheit |
| 42 | Auswerteeinheit |
| 44.1, 44.2 | Gegenelektroden |
| 46 | Dampfblase |
| 50 | Plasma |
| 52 | Gewebe |
| 60 | Verdampfungsphase |
| 62 | Zündphase |
| 64 | Gleichgewichtsphase |
| 66 | Initialphase |

**Patentansprüche**

1. Verfahren zum Betreiben eines Elektrochirurgie-Generators (12), wobei das Verfahren die folgenden Schritte umfasst:

   - Generieren einer Ausgangswechselspannung und Zuführen der Ausgangswechselspannung zu Ausgängen (18) des Elektrochirurgie-Generators (12), wobei für die Ausgangswechselspannung ein Ausgangswechselspannungsmaximalwert vorgegeben ist,
   - Bestimmen der Impedanz einer an den Ausgängen (18) anliegenden Last und Vergleichen der Impedanz mit einem Grenzwert, der so gewählt ist, dass dessen Unterschreiten das Bestehen einer Verdampfungsphase anzeigt,
   - wobei solange der Grenzwert unterschritten ist ein Ausgangswechselspannungsmaximalwert vorgegeben wird, der kleiner ist, als ein Ausgangsspannungsmaximalwert, der vorgegeben wird, wenn der Grenzwert überschritten wird, und
   - Regeln der Ausgangswechselspannung auf Grundlage des jeweils vorgegebenen Ausgangswechselspannungsmaximalwertes.

2. Verfahren zum Betreiben eines Elektrochirurgie-Generators (12) gemäß Anspruch 1, **dadurch gekennzeichnet, dass** der Ausgangswechselspannungsmaximalwert in Abhängigkeit der bestimmten Impedanz bei sich verändernder Impedanz nachgeführt wird, solange der Grenzwert unterschritten ist.

3. Verfahren zum Betreiben eines Elektrochirurgie-Generators (12), wobei das Verfahren die folgenden Schritte umfasst:

   - Generieren einer Ausgangswechselspannung und Zuführen der Ausgangswechselspannung zu Ausgängen (18) des Elektrochirurgie-Generators (12), wobei für die Ausgangswechselspannung ein Ausgangswechselspannungsmaximalwert vorgegeben ist,
   Bestimmen der Impedanz einer an den Ausgängen (18) anliegenden Last und Vergleich der Impedanz mit einem Grenzwert, der so gewählt ist, dass dessen Unterschreiten das Bestehen einer Verdampfungsphase anzeigt,
   - Erfassen eines Gleichspannungsanteils an der an den Ausgängen (18) anliegenden Ausgangswechselspannung,
   - wobei sobald der Grenzwert überschritten wird und kein Gleichspannungsanteil an der an den Ausgängen (18) anliegenden Ausgangswechselspannung erfasst wird, ein Ausgangswechselspannungsmaximalwert vorgegeben wird, der höher ist, als ein Ausgangsspannungsmaximalwert, der vorgegeben wird, wenn ein Gleichspannungsanteil erfasst wird, und
   - Regeln der Ausgangswechselspannung auf Grundlage des Ausgangswechselspannungsmaximalwertes.

4. Verfahren zum Betreiben eines Elektrochirurgie-Generators (12), wobei das Verfahren die Schritte gemäß der Ansprüche 1 und 3 umfasst.

5. Elektrochirurgie-Generator (12), der dazu konfiguriert ist, im Betrieb einen hochfrequenten Wechselstrom an ein elektrochirurgisches Instrument (14) für das Plasmaschneiden von Körpergewebe (52) abzugeben wobei der Elektrochirurgie-Generator (12) Ausgänge (18) zum Anschließen des elektrochirurgischen Instruments (14) aufweist, um ein im Betrieb an die Ausgänge (18) angeschlossenes elektrochirurgisches Instrument mit einem hochfrequenten Wechselstrom zu versorgen und, um eine Impedanz einer an die Ausgänge (18) angeschlossenen Last zu bestimmen wobei der Elektrochirurgie-Generator (12) außerdem eine Impedanzbestimmungseinheit und eine Spannungserfassungseinheit (40) sowie eine Ausgangsspannungsregeleinheit (36) aufweist, von denen

die Ausgangsspannungsregeleinheit (36) dazu konfiguriert ist, eine Ausgangswechselspannung des Elektrochirurgie-Generators (12) gemäß einem vorgegebenen Ausgangsspannungsmaximalwert zu steuern oder zu regeln, und

die Impedanzbestimmungseinheit dazu ausgebildet ist, eine Impedanz einer an den Ausgängen (18) des Elektrochirurgie-Generators (12) im Betrieb anliegenden Last zu bestimmen, **dadurch gekennzeichnet, dass**

die Ausgangsspannungsregeleinheit (36) dazu ausgebildet ist, die Ausgangswechselspannung in Abhängigkeit von einem Ausgangsspannungsmaximalwert zu steuern, der im Betrieb in Abhängigkeit von einem Ausgangswert der Impedanzbestimmungseinheit und/oder in Abhängigkeit von einem Ausgangwert der Spannungserfassungseinheit (40) so eingestellt wird, dass der Ausgangsspannungsmaximalwert in der Verdampfungsphase, die Teil der Initialphase ist, geringer ist, als der Ausgangsspannungsmaximalwert in einer späteren Gleichgewichtsphase.

6. Elektrochirurgie-Generator nach Anspruch 5, **dadurch gekennzeichnet, dass** die Ausgangsspannungsregeleinheit (36) dazu ausgebildet ist, die Ausgangswechselspannung in Abhängigkeit von einem Ausgangsspannungsmaximalwert zu steuern, der im Betrieb in Abhängigkeit von einem Ausgangswert der Impedanzbestimmungseinheit und/oder in Abhängigkeit von einem Ausgangswert der Spannungserfassungseinheit (40) so eingestellt wird, dass der Ausgangsspannungsmaximalwert in der Zündphase höher ist, als der Ausgangsspannungsmaximalwert in einer an die Zündphase anschließenden Gleichgewichtsphase.

7. Elektrochirurgie-Generator (12) nach wenigstens einem der Ansprüche 5 oder 6, **dadurch gekennzeichnet, dass** der Elektrochirurgie-Generator (12) dazu ausgebildet ist, die Verdampfungsphase anhand einer erfassten Impedanz zu detektieren.

8. Elektrochirurgie-Generator (12) nach Anspruch 7, **dadurch gekennzeichnet, dass** der Elektrochirurgie-Generator (12) dazu ausgebildet ist, ein Ende der Verdampfungsphase anhand eines Anstiegs der Impedanz an den Ausgängen des Elektrochirurgie-Generators über ein vorgegebenes Maß hinaus zu erfassen, wobei das vorgegebene Maß vorzugsweise aus einer zu Beginn der Verdampfungsphase von der Impedanzbestimmungseinheit erfassten Impedanz abgeleitet ist und vorzugsweise ein vorgegebenes Vielfaches dieser Impedanz beträgt.

9. Elektrochirurgie-Generator (12) nach wenigstens einem der Ansprüche 5 bis 8, **dadurch gekennzeichnet, dass** der Elektrochirurgie-Generator dazu ausgebildet ist, das Ende der Zündphase anhand eines Gleichspannungsanteils an einer an den Ausgängen des Elektrochirurgie-Generators anliegenden Spannung zu detektieren.

10. Elektrochirurgie-Generator (12) nach wenigstens einem der Ansprüche 5 bis 9, **dadurch gekennzeichnet, dass** die Ausgangsspannungsregeleinheit (36) dazu ausgebildet ist, während der Verdampfungsphase einen sich in Abhängigkeit von einem jeweils aktuellen Ausgangswert der Impedanzbestimmungseinheit und/oder der Spannungserfassungseinheit nachgeführten Ausgangsspannungsmaximalwert anzuwenden.

11. Elektrochirurgie-Generator (12) nach wenigstens einem der Ansprüche 5 bis 9, **dadurch gekennzeichnet, dass** die Ausgangsspannungsregeleinheit (36) dazu ausgebildet ist, während der Verdampfungsphase einen für die Verdampfungsphase fest vorgegebenen Ausgangsspannungsmaximalwert anzuwenden.

12. Elektrochirurgie-Generator (12) nach wenigstens einem der Ansprüche 5 bis 11, **dadurch gekennzeichnet, dass** der Elektrochirurgie-Generator (12) derart dimensioniert ist, dass er eine maximale Ausgangswechselspannung von mehr als 300 V und einen maximalen Ausgangswechselstrom von mehr als 4 A liefern kann.

13. Elektrochirurgie-System (10) mit einem Elektrochirurgie-Generator (12) nach wenigstens einem der Ansprüche 5 bis 12 und mit einem elektrochirurgischen Instrument (14), welches an Ausgänge (18) des Elektrochirurgie-Generators (12) angeschlossen ist oder angeschlossen werden kann, und welches eine aktive Elektrode (22) und wenigstens eine Gegenelektrode (44.1, 44.2) sowie wenigstens eine Flüssigkeitsleitung aufweist, die relativ zu der aktiven Elektrode (22) so angeordnet ist, dass die aktive Elektrode (22) im Betrieb mit einer elektrisch leitenden Flüssigkeit umspült werden kann.

14. Elektrochirurgie-System (10) nach Anspruch 13, **dadurch gekennzeichnet, dass** die aktive Elektrode als Loop-Elektrode oder als Button Elektrode ausgebildet ist und/oder dass das elektrochirurgische Instrument (14) ein Resektoskop ist.

**Claims**

1. The method of operating an electrosurgical generator (12), wherein the method comprises the following steps:

   - generating an AC output voltage and supplying the AC output voltage to outputs (18) of the electrosurgical generator (12), wherein a maximum AC output voltage value is predefined for the AC output voltage.
   - determining the impedance of a load present at the outputs (18) and comparing the impedance with a threshold value, which is defined such that an impedance falling below this threshold value indicates the occurrence of a vaporization phase.
   - wherein, as long as the impedance remains below the threshold value, a maximum AC output voltage value is preset that is lower than a maximum output voltage value that is preset when the threshold value is exceeded.
   - controlling the AC output voltage based on the respectively preset maximum AC output voltage value.

2. The method of operating an electrosurgical generator (12) according to claim 1, **characterized in that** the maximum AC output voltage value is updated depending on the determined impedance in a changing impedance, as long as the determined impedance remains below the threshold value.

3. The method of operating an electrosurgical generator (12), wherein the method comprises the following steps:

   - generating an AC output voltage and supplying the AC output voltage to outputs (18) of the electrosurgical generator (12), wherein a maximum AC output voltage value is predefined for the AC output voltage.
   - determining the impedance present at the outputs (18) and comparing the impedance with a threshold value, which is defined such that an impedance falling below this threshold value indicates the occurrence of a vaporization phase.
   - determining a DC offset in the AC output voltage present at the outputs (18),
   - wherein, as soon as the threshold value is exceeded and a DC offset is not detected in the AC output voltage present at the outputs (18), a maximum AC output voltage value is preset that is higher than a maximum output voltage value that is preset if a DC offset is detected, and
   - controlling the AC output voltage based on the maximum AC output voltage value.

4. The method of operating an electrosurgical generator (12), wherein the method comprises the steps according to claims 1 and 3.

5. Electrosurgical generator (12) that is configured to implement a method according to claim 1 and/or claim 3, and to supply, during operation, a high-frequency alternating current to an electrosurgical instrument (14) for plasma cutting of body tissue (52), wherein the electrosurgical generator (12) has outputs (18) for connecting an electrosurgical instrument (14) to supply an electrosurgical instrument connected to the outputs (18) during operation with a high-frequency alternating current, and for determining the impedance of a load connected to the outputs (18);

   wherein the electrosurgical generator (12) further features an impedance measuring unit and a voltage measuring unit (40) as well as an output voltage control unit (36), of which
   the output voltage control unit (36) is configured to control or regulate an AC output voltage of the electrosurgical generator (12) in accordance with a predefined maximum output voltage value, and
   the impedance measuring unit is designed to determine an impedance of a load present at the outputs (18) of the electrosurgical generator (12) during operation;
   **characterized in that**
   the output voltage control unit (36) is designed to control the AC output voltage depending on a maximum output voltage value that is set depending on an output value of the impedance measuring unit and/or depending on an output value of the voltage measuring unit (40), such that the maximum output voltage value during the vaporization phase being part of the initial phase is lower than the maximum output voltage value during a later equilibrium phase.

6. Electrosurgical generator according to claim 5, **characterized in that** the output voltage control unit (36) is designed to control the AC output voltage depending on a maximum output voltage value that is set during operation depending on an output value of the impedance measuring unit and/or depending on an output value of the voltage measuring unit (40), such that the maximum output voltage value is higher during an ignition phase than the maximum output voltage value during an equilibrium phase occurring subsequently to the ignition phase.

7. Electrosurgical generator (12) according to at least one of claims 5 or 6, **characterized in that** the electrosurgical generator (12) is designed to detect the vaporization phase based on a measured impedance.

8. Electrosurgical generator (12) according to claim 7, **characterized in that** the electrosurgical generator (12) is designed to detect the end of the vapor phase based on an impedance increase at the outputs of the electrosurgical generator beyond a predefined value, wherein the predefined value is preferably derived from the impedance measured by the impedance measuring unit at the start of the vaporization phase, and is preferably a predefined multiple of this impedance.

9. Electrosurgical generator (12) according to at least one of claims 7 to 8, **characterized in that** the electrosurgical generator is designed to detect the end of the ignition phase based on a DC offset in the voltage present at the outputs of the electrosurgical generator.

10. Electrosurgical generator (12) according to at least one of claims 5 to 9, **characterized in that** the output voltage control unit (36) is designed to apply, during the vaporization phase, a maximum output voltage value updated depending on a respectively current output value of the impedance measuring unit and/or the voltage measuring unit.

11. Electrosurgical generator (12) according to at least one of claims 5 to 9, **characterized in that** the output voltage control unit (36) is designed to apply, during the vaporization phase, a maximum output voltage value that is predefined for the vaporization.

12. Electrosurgical generator (12) according to at least one of claims 5 to 11, **characterized in that** the electrosurgical generator (12) is dimensioned in such a way that it can supply a maximum AC output voltage of more than 300 V and a maximum AC output current of more than 4 A.

13. Electrosurgical system (10) with an electrosurgical generator (12) according to at least one of claims 5 to 12, and with an electrosurgical instrument (14) that is or can be connected to outputs (18) of the electrosurgical generator (12), and that has an active electrode (22) and at least one return electrode (44.1, 44.2) as well as at least one fluid line that is arranged relative to the active electrode (22) in such a way that the active electrode (22) can be surrounded by an electroconductive fluid during operation.

14. Electrosurgical system (10) according to claim 13, **characterized in that** the active electrode is designed as a loop electrode or a button electrode and/or wherein the electrosurgical instrument (14) is a resectoscope.

**Revendications**

1. Procédé pour faire fonctionner un générateur (12) d'électrochirurgie, dans lequel le procédé comprend les stades suivants :

   - création d'une tension alternative de sortie et application de la tension alternative de sortie à des sorties (18) du générateur (12) d'électrochirurgie, dans lequel il est prescrit à la tension alternative de sortie une valeur maximum de tension alternative de sortie,
   - détermination de l'impédance d'une charge s'appliquant aux sorties (18) et comparaison de l'impédance à une valeur limite, qui est choisie de manière à ce que passer en dessous de celle-ci indique l'existence d'une phase d'évaporation,
   - dans lequel, tant que l'on est en dessous de la valeur limite, on prescrit une valeur maximum de la tension alternative de sortie, qui est plus basse que la valeur maximum de la tension de sortie, qui est prescrite, lorsque la valeur limite est dépassée, et
   - réglage de la tension alternative de sortie sur la base de la valeur maximum de la tension alternative de sortie prescrite respectivement.

2. Procédé pour faire fonctionner un générateur (12) d'électrochirurgie suivant la revendication 1, **caractérisé en ce que** l'on suit la valeur maximum de la tension alternative de sortie en fonction de l'impédance déterminée, lorsque l'impédance se modifie, tant que l'on est en dessous de la valeur limite.

3. Procédé pour faire fonctionner un générateur (12) d'électrochirurgie, dans lequel le procédé comprend les stades suivants :

   - création d'une tension alternative de sortie et application de la tension alternative de sortie à des sorties (18) du générateur (12) d'électrochirurgie, dans lequel il est prescrit à la tension alternative de sortie une valeur maximum de tension alternative de sortie,
   - détermination de l'impédance d'une charge s'appliquant aux sorties (18) et comparaison de l'impédance à une valeur limite, qui est choisie de manière à ce que passer en dessous de celle-ci indique l'existence d'une phase d'évaporation,

- détection d'une composante de la tension continue sur la tension alternative de sortie s'appliquant aux sorties (18),

- dans lequel, tant que l'on est au-dessus de la valeur limite et que l'on ne détecte pas de composante de tension continue sur la tension alternative de sortie s'appliquant aux sorties (18), on prescrit une valeur maximum de la tension alternative de sortie, qui est plus grande qu'une valeur maximum de tension de sortie, qui est prescrite lorsque l'on détecte une composante de tension continue, et

- réglage de la tension alternative de sortie sur la base de la valeur maximum de la tension alternative de sortie.

4. Procédé pour faire fonctionner un générateur (12) d'électrochirurgie, dans lequel le procédé comprend les stades suivant les revendications 1 et 3.

5. Générateur (12) d'électrochirurgie, qui est configuré pour donner, en fonctionnement, un courant alternatif de haute fréquence à un instrument (14) électrochirurgical pour la coupe au plasma de tissu (52) du corps, dans lequel le générateur (12) d'électrochirurgie a des sorties (18) de raccordement de l'instrument (14) électrochirurgical afin d'alimenter en courant alternatif de haute fréquence un instrument électrochirurgical raccordé aux sorties (18) en fonctionnement et afin de déterminer une impédance d'une charge raccordée aux sorties (18),

    dans lequel le générateur (12) d'électrochirurgie a, en outre, une unité de détermination d'impédance et une unité (40) de détection de tension, ainsi qu'une unité (36) de réglage de la tension de sortie, dont

    l'unité (36) de réglage de tension de sortie est configurée pour commander ou régler une tension alternative de sortie du générateur (12) d'électrochirurgie suivant une valeur maximum prescrite de la tension de sortie, et

    l'unité de détermination d'impédance est constituée pour déterminer une impédance d'une charge s'appliquant en fonctionnement aux sorties (18) du générateur (12) d'électrochirurgie, **caractérisé en ce que**

    l'unité (36) de réglage de la tension de sortie est constituée pour commander la tension alternative de sortie en fonction d'une valeur maximum de la tension de sortie, qui est réglée en fonctionnement, en fonction d'une valeur de sortie de l'unité de détermination de l'impédance et/ou en fonction d'une valeur de sortie de l'unité (40) de détection de la tension, de manière à ce que la valeur maximum de la tension de sortie dans la phase d'évaporation, qui est une partie de la phase initiale, soit plus basse que la valeur maximum de la tension de sortie d'une phase d'équilibre ultérieure.

6. Générateur d'électrochirurgie suivant la revendication 5, **caractérisé en ce que**
l'unité (36) de réglage de la tension de sortie est constituée pour commander la tension alternative de sortie en fonction d'une valeur maximum de la tension de sortie, qui est réglée en fonctionnement en fonction d'une valeur de sortie de l'unité de détermination de l'impédance et/ou en fonction d'une valeur de sortie de l'unité (40) de détection de la tension, de manière à ce que la valeur maximum de la tension de sortie soit plus grande dans la phase d'amorçage que la valeur maximum de la tension de sortie dans une phase d'équilibre faisant suite à la phase d'amorçage.

7. Générateur (12) d'électrochirurgie suivant au moins l'une des revendications 5 ou 6, **caractérisé en ce que** le générateur (12) d'électrochirurgie est constitué pour détecter la phase d'évaporation, à l'aide de la détection de l'impédance.

8. Générateur (12) d'électrochirurgie suivant la revendication 7, **caractérisé en ce que** le générateur (12) d'électrochirurgie est constitué pour détecter une fin de la phase d'évaporation, à l'aide d'une augmentation de l'impédance sur les sorties du générateur d'électrochirurgie au-delà d'une mesure donnée à l'avance, dans lequel la mesure donnée à l'avance est déduite, de préférence, d'une impédance détectée au début de la phase d'évaporation par l'unité de détermination d'impédance et représente de préférence un multiple donné à l'avance de cette impédance.

9. Générateur (12) d'électrochirurgie suivant au moins l'une des revendications 5 à 8, **caractérisé en ce que** le générateur d'électrochirurgie est constitué pour détecter la fin de la phase d'amorçage, à l'aide d'une composante de tension continue sur une tension s'appliquant aux sorties du générateur d'électrochirurgie.

10. Générateur (12) d'électrochirurgie suivant au moins l'une des revendications 5 à 9, **caractérisé en ce que** l'unité (36) de réglage de la tension de sortie est constituée pour appliquer, pendant la phase d'évaporation, une valeur maximum de la tension de sortie suivie en fonction d'une valeur de sortie en cours respective de l'unité de détermination d'impédance et/ou de l'unité de détection de la tension.

11. Générateur (12) d'électrochirurgie suivant au moins l'une des revendications 5 à 9, **caractérisé en ce**

**que** l'unité (36) de réglage de la tension de sortie est constituée pour appliquer, pendant la phase d'évaporation, une valeur maximum de la tension de sortie donnée à l'avance de manière fixe pour la phase d'évaporation.

12. Générateur (12) d'électrochirurgie suivant au moins l'une des revendications 5 à 11, **caractérisé en ce que** le générateur (12) d'électrochirurgie est dimensionné, de manière à pouvoir donner une tension alternative de sortie maximum de plus de 300 V et un courant alternatif de sortie maximum de plus de 4 A**.**

13. Système (10) d'électrochirurgie comprenant un générateur (12) d'électrochirurgie suivant au moins l'une des revendications 5 à 12 et comprenant un instrument (14) électrochirurgical, qui est raccordé aux sorties (18) du générateur (12) d'électrochirurgie ou qui peut l'être et qui a une électrode (22) active et au moins une contre-électrode (44.1, 44.2) ainsi qu'au moins un conduit pour du liquide, qui est disposé, par rapport à l'électrode (22) active, de manière à ce qu'en fonctionnement l'électrode (22) active puisse être baignée dans un liquide conducteur de l'électricité.

14. Système (10) d'électrochirurgie suivant la revendication 13, **caractérisé en ce que** l'électrode active est constituée sous la forme d'une électrode loop ou d'une électrode button et/ou **en ce que** l'instrument (14) électrochirurgical est un résectoscope.

Fig. 1

EP 3 884 894 B1

Fig. 2

Fig. 3

EP 3 884 894 B1

Fig. 4

22''''

a)           b)           c)

Fig. 5

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2014236142 A1